# EUROPEAN PATENT APPLICATION

(11) **EP 4 830 694 A1**
(43) Date of publication of application: **28.10.2026**
(21) Application number: 24907206.7
(22) Date of filing: 06.12.2024
(51) Int. Cl.: A61K 8/81, A61K 8/02, A61K 8/84, A61Q 1/00, A61Q 5/00, A61Q 17/04, A61Q 19/00

(54) **FEEL IMPROVER AND COMPOSITION**

(30) Priority: 19.12.2023 JP 2023213459; 29.02.2024 JP 2024029856
(71) Applicant: Nippon Shokubai Co., Ltd., Osaka-shi, Osaka 541-0043 (JP)
(72) Inventor: NISHINA, Akira, Suita-shi, Osaka 564-0034 (JP); DEGUCHI, Junya, Suita-shi, Osaka 564-0034 (JP)
(74) Representative: dompatent
(86) International application number: PCT/JP2024/043272
(87) International publication number: WO 2025/134820

(57) **Abstract**

Provided is a texture improver excellent in texture improving properties when used in blending into a cosmetic together with a powder. A texture improver contains a primary to tertiary amino group-containing polymer compound. The primary to tertiary amino group-containing polymer compound includes: preferably a copolymer containing a structural unit (a) derived from a primary to tertiary amino group-containing monomer (A) and a structural unit (b) derived from a hydrophobic monomer (B); and/or a polyalkyleneimine. The hydrophobic monomer (B) preferably contains a (meth)acrylic acid ester.

## Description

### Technical Field

The present invention relates to a texture improver. The present invention also relates to a composition, preferably a composition for use in a cosmetic.

### Background Art

Powders are typically blended into makeup cosmetics such as foundation, eye shadow, and blush, sunscreen cosmetics, milky lotions, and the like. For the purpose of improving adhesion to the skin and texture of powders, surface-treated powders obtained by coating surfaces of pigment powders with various compounds are generally used as powders to be blended in cosmetics. As such surface-treated powders, for example, those disclosed in Patent Documents 1 and 2 are known.

### Citation List

### Patent Document

Patent Document 1: JP 2014-101279 A
Patent Document 2: JP 2006-342322 A

### Summary of Invention

### Technical Problem

However, when the surface-treated powders disclosed in Patent Documents 1 and 2 are used, it is necessary to perform a surface treatment on powders in advance, and only specific powders can be used.

Accordingly, an object of the present invention is to provide a texture improver that is excellent in texture improving properties when used in blending into a cosmetic together with a powder.

### Solution to Problem

As a result of intensive studies to achieve the above-described object, the present inventors have found that a texture improver containing a specific polymer compound is excellent in texture improving properties when used in blending into a cosmetic together with a powder. The present invention has been completed based on these findings.

That is, the present invention provides a texture improver containing a primary to tertiary amino group-containing polymer compound.

The primary to tertiary amino group-containing polymer compound preferably includes: a copolymer containing a structural unit (a) derived from a primary to tertiary amino group-containing monomer (A) and a structural unit (b) derived from a hydrophobic monomer (B); and/or a polyalkyleneimine.

The hydrophobic monomer (B) preferably contains a (meth)acrylic acid ester.

The polyalkyleneimine is preferably polyethyleneimine.

The present invention also provides an inorganic powder having the above-described texture improver present on a surface of the inorganic powder.

The present invention also provides a composition containing the above-described texture improver and an inorganic powder.

In addition, the present invention provides a composition containing a primary to tertiary amino group-containing polymer compound and an inorganic powder.

The primary to tertiary amino group-containing polymer compound preferably includes: a copolymer containing a structural unit (a) derived from a primary to tertiary amino group-containing monomer (A) and a structural unit (b) derived from a hydrophobic monomer (B); and/or a polyalkyleneimine.

The hydrophobic monomer (B) preferably contains a (meth)acrylic acid ester.

The polyalkyleneimine is preferably polyethyleneimine.

The composition is preferably a cosmetic composition.

The present invention also provides a method for producing an inorganic powder, the inorganic powder having the texture improver present on a surface of the inorganic powder, the method including a mixing step of mixing the texture improver and an inorganic powder in a solvent.

The production method preferably includes a step of removing the solvent after the mixing step.

The production method preferably includes a pulverization step of pulverizing the inorganic powder after the mixing step.

### Advantageous Effects of Invention

The texture improver of the present invention is excellent in texture improving properties when used in blending into a cosmetic together with a powder.

### Brief Description of Drawings

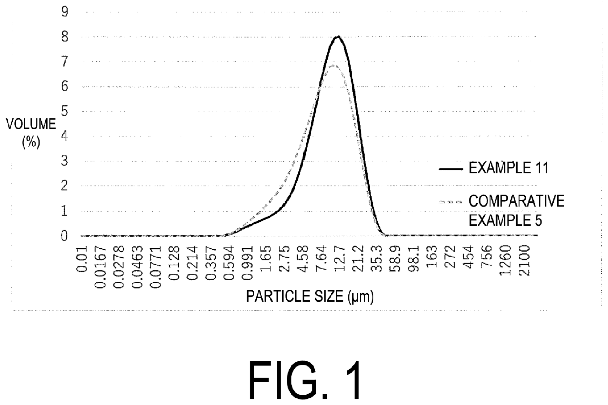

FIG. 1 illustrates particle size distributions of Example 11 and Comparative Example 5.

### Description of Embodiments

### Texture Improver

The texture improver of the present invention contains at least a primary to tertiary amino group-containing polymer compound. By blending the texture improver, together with a powder, into a composition to be applied to the skin, the texture of the composition on the skin can improve. The texture improver of the present invention may contain only one type of the primary to tertiary amino group-containing polymer compound, or may contain two or more types thereof.

The polymer compound is presumed to act as a cationic polymer by having a primary to tertiary amino group, and can improve the texture when blended, together with a powder, into a cosmetic and applied to the skin. In addition, while a polymer compound having a quaternary ammonium group has a safety concern, the primary to tertiary amino group-containing polymer compound does not form a quaternary ammonium group in the composition, and thus has a relatively small concern about the safety.

The primary to tertiary amino group-containing polymer compound preferably includes: a resin containing a structural unit (a) derived from a primary to tertiary amino group-containing monomer (A); and/or a polyalkyleneimine.

### Resin Containing Structural Unit (a) Derived from Monomer (A)

The resin containing the structural unit (a) derived from the primary to tertiary amino group-containing monomer (A) is preferably a copolymer containing the structural unit (a) derived from the primary to tertiary amino group-containing monomer (A) and a structural unit (b) derived from a hydrophobic monomer (B). Due to the primary to tertiary amino group-containing monomer (A) being contained as a monomer component, improvement in the adhesion of the composition containing the inorganic powder to the skin can be expected, in addition to the effect of improving the texture on the skin. When the hydrophobic monomer (B) is contained as a monomer component, a hydrophobic group in the hydrophobic monomer (B) is adsorbed on the surface of the inorganic powder, and improvement in dispersion stability of the inorganic powder and improvement in texture improving effect can be expected.

The "structural unit (a) derived from the primary to tertiary amino group-containing monomer (A)" may be formed by another production method as long as it has the same structure as a structural unit formed by polymerization of the primary to tertiary amino group-containing monomer (A). The same applies to the "structural unit (b) derived from the hydrophobic monomer (B)" and the "structural unit (e) derived from an additional monomer (E)" described later. For example, when the primary to tertiary amino group-containing monomer (A) is N,N-dimethylaminoethyl methacrylate, the structural unit (a) is a structural unit represented by -CH₂-C(CH₃)(COCH₂CH₂-N(CH₃)₂)-.

Examples of the primary to tertiary amino group-containing monomer (A) include a compound having at least one ethylenically unsaturated group and at least one primary to tertiary amino group, and a neutralized product of the primary to tertiary amino group in the compound with an acid. The primary to tertiary amino group is preferably a group represented by the following Formula (1).

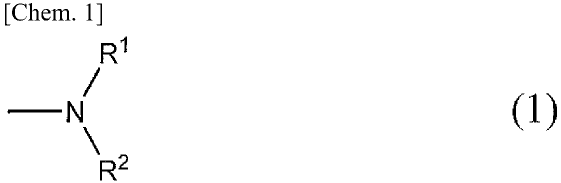

[In Formula (1), R¹ and R² are the same or different and each represent a hydrogen atom or a hydrocarbon group.]

The number of carbon atoms of the hydrocarbon group is preferably from 1 to 12, more preferably from 1 to 10, still more preferably from 1 to 8, yet still more preferably from 1 to 5, yet still more preferably from 1 to 2, and particularly preferably 1.

The hydrocarbon group may be a linear or branched hydrocarbon group (aliphatic hydrocarbon group), or may be an alicyclic hydrocarbon group having a cyclic structure or an aromatic hydrocarbon group, but is preferably a linear or branched hydrocarbon group.

The hydrocarbon group is preferably an alkyl group, an alkenyl group, or an aryl group, more preferably an alkyl group or an alkenyl group, and still more preferably an alkyl group. Examples of the alkyl group and the alkenyl group include groups that are exemplified and described in the (meth)acrylic acid ester below.

Examples of the aryl group include a phenyl group, a benzyl group, a methylphenyl group, a 1-methoxy-4-methylphenyl group, an ethylphenyl group, a propylphenyl group, a butylphenyl group, a butylmethylphenyl group, a dimethylphenyl group, a diethylphenyl group, a biphenyl group, a naphthyl group, a naphthylmethyl group, and a naphthylethyl group.

At least one of R¹ and R² described above is preferably a hydrocarbon group (particularly, a hydrocarbon group having from 1 to 12 carbon atoms), and both R¹ and R² are more preferably hydrocarbon groups (particularly, hydrocarbon groups having from 1 to 12 carbon atoms). That is, among the primary to tertiary amino groups, a tertiary amino group is preferable.

As described above, a neutralized product obtained by neutralizing the primary to tertiary amino group with an acid can also be used. The neutralized product of the primary to tertiary amino group is preferably a neutralized product having a structure represented by the following Formula (1').

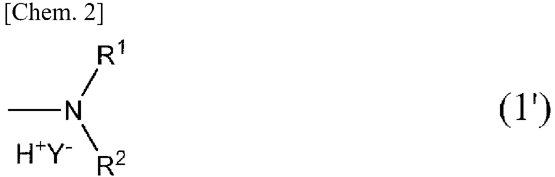

[In Formula (1'), R¹ and R² are the same or different and each represent a hydrogen atom or a hydrocarbon group. Y⁻ represents an anion.]

Preferred aspects of R¹ and R² in the above-described Formula (1') are the same as those of R¹ and R² in the above-described Formula (1).

Y⁻ in the above-described Formula (1') is not particularly limited, and examples thereof include halide ions such as a chlorine ion, a bromine ion, and an iodine ion; alkyl sulfate ions such as a methyl sulfate ion; and organic acid ions such as an acetate ion. Among them, an organic acid ion is preferable.

The primary to tertiary amino group-containing monomer (A) is preferably a monomer represented by the following Formula (1-1) or a monomer represented by the following Formula (1-2).

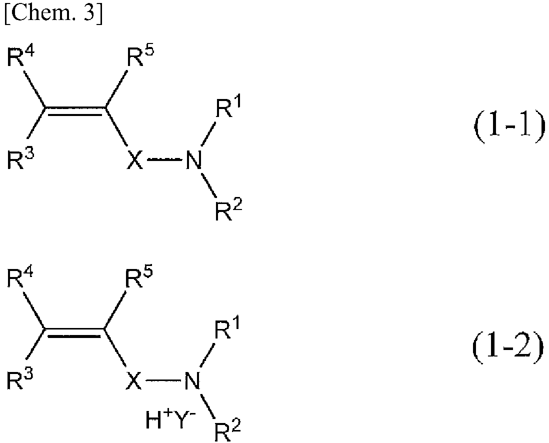

[In Formula (1-1), R³ to R⁵ are the same or different and each represent a hydrogen atom or an alkyl group having from 1 to 5 carbon atoms. X represents a direct bond or a divalent linking group. R¹ and R² are the same as R¹ and R² in the above-described Formula (1).]

[In Formula (1-2), R³ to R⁵ are the same or different and each represent a hydrogen atom or an alkyl group having from 1 to 5 carbon atoms. X represents a direct bond or a divalent linking group. R¹, R², and Y⁻ are the same as R¹, R², and Y⁻ in the above-described Formula (1').]

The alkyl group having from 1 to 5 carbon atoms in R⁵ described above is preferably a methyl group. R⁵ described above is preferably a hydrogen atom or a methyl group. From the viewpoint of hydrolysis resistance, R⁵ is more preferably a methyl group. R³ and R⁴ described above are preferably hydrogen atoms.

The divalent linking group in X is not particularly limited, and examples thereof include an alkylene group having from 1 to 12 carbon atoms, a group represented by the following Formula (2), a group represented by the following Formula (3), and a group represented by the following Formula (4). 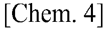 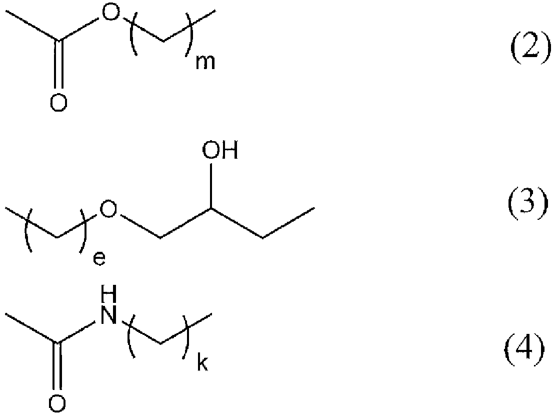 In Formula (2), m represents an integer from 0 to 12, preferably from 1 to 8, and more preferably from 1 to 5.

In Formula (3), e represents an integer from 0 to 4, preferably from 1 to 3, and more preferably from 1 to 2.

In Formula (4), k represents an integer from 1 to 10, preferably from 1 to 8, and more preferably from 1 to 5.

Especially, X described above is preferably a group represented by Formula (2).

As the primary to tertiary amino group-containing monomer (A), a monomer represented by the above-described Formula (1), in which R⁵ in the above-described Formula (1-1) is a methyl group and X in the above-described Formula (1-1) is a group represented by the above-described Formula (2), is particularly preferable.

Specific examples of the primary to tertiary amino group-containing monomer (A) include N,N-dialkylamino group-containing (meth)acrylates such as N,N-dimethylaminoethyl (meth)acrylate, N,N-diethylaminoethyl (meth)acrylate, N,N-dimethylaminopropyl (meth)acrylate, and N,N-diethylaminopropyl (meth)acrylate; N,N-dialkylamino group-containing (meth)acrylamides such as N,N-dimethylaminoethyl (meth)acrylamide, N,N-diethylaminoethyl (meth)acrylamide, N,N-dimethylaminopropyl (meth)acrylamide, and N,N-diethylaminopropyl (meth)acrylamide; monoalkylamino group-containing (meth)acrylates such as monomethylaminoethyl (meth)acrylate, monoethylaminoethyl (meth)acrylate, monomethylaminopropyl (meth)acrylate, monoethylaminopropyl (meth)acrylate, and 2-(tert-butylamino)ethyl (meth)acrylate; monoalkylamino group-containing (meth)acrylamides such as monomethylaminoethyl (meth)acrylamide, monoethylaminoethyl (meth)acrylamide, monomethylaminopropyl (meth)acrylamide, and monoethylaminopropyl (meth)acrylamide; esters of (meth)acrylic acid and alkanolamine such as 2-aminoethyl (meth)acrylate; N,N-diallylmethylamine; allylamine; and addition reaction products of an unsaturated monomer having a cyclic ether-containing group having from 2 to 8 carbon atoms and an amine compound having from 1 to 24 carbon atoms, such as 1-allyloxy-3-dibutylamino-2-ol and 1-allyloxy-3-diethanolamino-2-ol.

The number of carbon atoms in the amine compound having from 1 to 24 carbon atoms is preferably from 1 to 20, and more preferably from 1 to 16. Examples of the amine compound having from 1 to 24 carbon atoms include primary amines and secondary amines, and examples thereof include (di)alkylamines having from 1 to 24 carbon atoms, (di)alkanolamines having from 1 to 24 carbon atoms, and alkylalkanolamines having from 1 to 24 carbon atoms.

Examples of the (di)alkylamine having from 1 to 24 carbon atoms include methylamine, ethylamine, propylamine, butylamine, dimethylamine, diethylamine, dipropylamine, dibutylamine, pentylamine, dipentylamine, hexylamine, dihexylamine, heptylamine, diheptylamine, octylamine, dioctylamine, dodecylamine, and didodecylamine.

Examples of the (di)alkanolamine having from 1 to 24 carbon atoms include methanolamine, ethanolamine, propanolamine, butanolamine, dimethanolamine, diethanolamine, dipropanolamine, dibutanolamine, and hexanolamine.

Examples of the alkylalkanolamine having from 1 to 24 carbon atoms include methylethanolamine.

Among them, the primary to tertiary amino group-containing monomer (A) is preferably an N,N-dialkylamino group-containing (meth)acrylate or an N,N-dialkylamino group-containing (meth)acrylamide, and more preferably N,N-dimethylaminoethyl (meth)acrylate, N,N-diethylaminoethyl (meth)acrylate, or N,N-dimethylaminopropyl (meth)acrylamide.

A content proportion of the structural unit (a) derived from the primary to tertiary amino group-containing monomer (A) in the copolymer is preferably from 36 to 99.9 mass%, more preferably from 40 to 99 mass%, still more preferably from 45 to 98 mass%, yet still more preferably from 50 to 95 mass%, yet still more preferably from 52 to 90 mass%, and particularly preferably from 55 to 80 mass%, per 100 mass% of a total amount of structural units constituting the copolymer.

The hydrophobic monomer (B) is a monomer in which a homopolymer obtained by homopolymerization has a solubility parameter of 15 or less. Even if the solubility parameter is 15 or less, the monomer corresponding to the primary to tertiary amino group-containing monomer (A) is treated as the primary to tertiary amino group-containing monomer (A) and does not correspond to the hydrophobic monomer (B). Here, the solubility parameter is a value calculated by the method described in "POLYMER ENGINEERING AND SCIENCE" (1974, Vol. 14, No. 2), pages 147 to 154. The method is outlined below. The solubility parameter (δ) of the homopolymer (cal/cm³)^{1/2} is calculated by the following calculation method based on an evaporation energy (Δei) and a molar volume (Δvi) of a constituent unit forming the polymer.
δ = (Δei/Δvi)^{1/2} (cal/cm³)^{1/2}

The solubility parameter of the homopolymer obtained by polymerizing the hydrophobic monomer (B) alone is preferably 14 or less, more preferably 13 or less, and still more preferably 12 or less. The solubility parameter is usually 5 or more.

Examples of the hydrophobic monomer (B) include esters of (meth)acrylic acid and alcohols which may have a substituent ((meth)acrylic acid esters); unsaturated monocarboxylic acids (salts) such as (meth)acrylic acid, crotonic acid, α-allyloxyacrylic acid, and salts thereof; aromatic vinyl-based monomers such as styrene; olefin-based monomers such as ethylene and propylene; esters of unsaturated alcohols and carboxylic acids such as vinyl acetate; vinyl halides such as vinyl chloride; alkyl vinyl ethers such as methyl vinyl ether and ethyl vinyl ether; addition reaction products of unsaturated monomers having a cyclic ether-containing group having from 2 to 8 carbon atoms and alcohols having from 1 to 20 carbon atoms, such as 1-allyloxy-3-butoxypropan-2-ol; alkylene oxide adducts of unsaturated alcohols having from 2 to 20 carbon atoms such as ethylene oxide adducts of allyl alcohol, ethylene oxide adducts of methallyl alcohol, and ethylene oxide adducts of isoprenol, and terminally hydrophobically modified products thereof; and cyclic vinyl-based monomers such as N-vinylpyrrolidone. As the hydrophobic monomer (B), among those having a solubility parameter of 15 or less, those having an alkyl group having from 2 to 12 carbon atoms are preferable. This makes hydrophobicity of the hydrophobic monomer (B) fall within a more suitable range, the hydrophobic group in the hydrophobic monomer (B) is adsorbed on a surface of the inorganic powder, and improvement in dispersion stability of the inorganic powder and improvement in texture improving effect can be expected.

Examples of the unsaturated monocarboxylic acid salt include metal salts. Examples of the metal of the metal salt include alkali metals such as lithium, sodium, and potassium.

Examples of the substituent which the (meth)acrylic acid ester may have include hydroxyl groups; alkoxy groups having from 1 to 18 carbon atoms such as a methoxy group and an ethoxy group; oxo group-containing groups such as an oxyalkylene group, a sulfonic acid group, and a phosphoric acid group; halogeno groups such as a fluoro group; epoxy groups such as a glycidyl group; and carbonyl groups such as an aldehyde group.

Examples of the (meth)acrylic acid ester having no substituent include cycloalkyl (meth)acrylates such as methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, sec-butyl (meth)acrylate, 2-ethylhexyl (meth)acrylate, n-octyl (meth)acrylate, tridecyl (meth)acrylate, and cyclohexyl (meth)acrylate; and alkyl (meth)acrylates such as n-lauryl (meth)acrylate, dodecyl (meth)acrylate, stearyl (meth)acrylate, and isobornyl methacrylate.

Examples of the (meth)acrylic acid ester having a hydroxyl group include hydroxyl group-containing (meth)acrylates in which an ester group has from 1 to 18 carbon atoms, such as 2-hydroxyethyl (meth)acrylate, 2-hydroxypropyl (meth)acrylate, 3-hydroxypropyl (meth)acrylate, 2-hydroxybutyl (meth)acrylate, and 4-hydroxybutyl (meth)acrylate.

Examples of the (meth)acrylic acid ester having an alkoxy group include alkoxyalkyl (meth)acrylates such as methoxyethyl (meth)acrylate, methoxypropyl (meth)acrylate, methoxybutyl (meth)acrylate, ethoxybutyl (meth)acrylate, and trimethylolpropane tripropoxy (meth)acrylate.

Examples of the (meth)acrylic acid ester having an oxo group include (di)ethylene glycol (methoxy) (meth)acrylates such as ethylene glycol (meth)acrylate, ethylene glycol methoxy (meth)acrylate, diethylene glycol (meth)acrylate, and diethylene glycol methoxy (meth)acrylate; alkoxypolyalkylene glycol (meth)acrylates having the number of repetitions of alkylene glycol of 1 to 100 such as alkoxypolyethylene glycol methacrylate (ANTOX LMA-10); sulfopropyl (meth)acrylates; and oxo group-containing (meth)acrylates such as (meth)acryloyloxyethyl phosphate.

Examples of the (meth)acrylic acid ester having a fluoro group include fluoro group-containing alkyl (meth)acrylates such as fluoro group-containing alkyl (meth)acrylates containing an ester group having from 2 to 6 carbon atoms, such as trifluoroethyl (meth)acrylate, tetrafluoropropyl (meth)acrylate, and octafluoropentyl (meth)acrylate.

Examples of the (meth)acrylic acid ester having an epoxy group include epoxy group-containing (meth)acrylates such as glycidyl (meth)acrylate, α-methylglycidyl (meth)acrylate, and glycidyl allyl ether.

Examples of the (meth)acrylic acid ester having a carbonyl group include carbonyl group-containing (meth)acrylates such as acetonyl (meth)acrylate, diacetone (meth)acrylate, 2-hydroxypropyl (meth)acrylate acetylacetate, butanediol-1,4-acrylate acetylacetate, 2-(acetoacetoxy)ethyl (meth)acrylate, and (meth)acryloyloxyalkylpropenal.

The hydrophobic monomer (B) preferably contains a (meth)acrylic acid ester (hereinafter, sometimes referred to as "monomer (B1)").

The (meth)acrylic acid ester is preferably a compound represented by the following Formula (5).

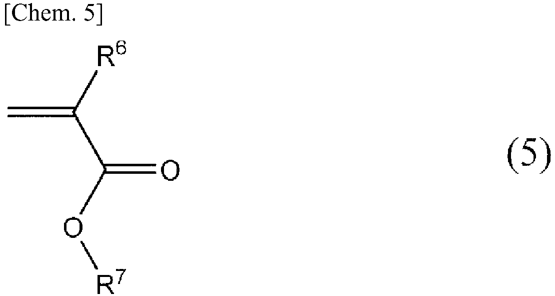

[In Formula (5), R⁶ represents a hydrogen atom or a methyl group. R⁷ represents a hydrocarbon group having from 1 to 30 carbon atoms.]

The number of carbon atoms of the hydrocarbon group in R⁷ described above is preferably from 1 to 20, more preferably from 1 to 16, still more preferably from 1 to 12, yet still more preferably from 1 to 8, and particularly preferably from 2 to 8. When the hydrocarbon group has from 1 to 20 carbon atoms, water solubility and viscosity of the polymer can be in suitable ranges, and the polymer is excellent in handling. When the hydrocarbon group has from 1 to 12 carbon atoms, the polymer is easily produced, and, further, the hydrophobicity is in a suitable range. Thus, the hydrophobic group in the hydrophobic monomer (B) is adsorbed on a surface of the inorganic powder, and improvement in dispersion stability of the inorganic powder and improvement in texture improving effect can be expected.

Examples of the hydrocarbon group in R⁷ described above include chain hydrocarbon groups such as an alkyl group, an alkenyl group, and an alkynyl group, aromatic hydrocarbon groups, and cyclic hydrocarbon groups such as a cycloalkyl group and a cycloalkenyl group. The hydrocarbon group may have a branched chain, and the number of carbon atoms of the hydrocarbon group in the case of having a branched chain means the total number of carbon atoms of the main chain and the branched chain.

Examples of the alkyl group include a methyl group, an ethyl group, a propyl group, an isopropyl group, a butyl group, an isobutyl group, a hexyl group, a heptyl group, a 2-ethylhexyl group, an octyl group, a nonyl group, a decyl group, a dodecyl group, a stearyl group, and an icosyl group. Examples of the alkenyl group include a vinyl group, an allyl group, a 1-butenyl group, a 2-butenyl group, a pentenyl group, a hexenyl group, a heptenyl group, an octenyl group, a nonenyl group, a decenyl group, a dodecenyl group, an octadecenyl group, and an icosenyl group. Examples of the alkynyl group include an ethynyl group, a 1-propynyl group, a 2-propynyl group, a butynyl group, a pentynyl group, a hexynyl group, a heptynyl group, an octynyl group, a nonynyl group, a decynyl group, a dodecynyl group, an octadecynyl group, and an icosynyl group.

Examples of the aromatic hydrocarbon group include a phenyl group, a benzyl group, a tolyl group, and an o-xylyl group. Examples of the cycloalkyl group include a cyclopropyl group, a cyclobutyl group, a cyclopentyl group, and a cyclohexyl group. Examples of the cycloalkenyl group include a cyclopropenyl group, a cyclobutenyl group, a cyclopentenyl group, and a cyclohexenyl group.

Among them, the hydrocarbon group is preferably an alkyl group or an alkenyl group, and more preferably an alkyl group. That is, for the (meth)acrylic acid ester, preferable is a (meth)acrylic acid alkyl ester (alkyl (meth)acrylate).

For the (meth)acrylic acid alkyl ester, preferable are methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, sec-butyl (meth)acrylate, 2-ethylhexyl (meth)acrylate, and n-octyl (meth)acrylate; and methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, sec-butyl (meth)acrylate, and 2-ethylhexyl (meth)acrylate are more preferable.

A content proportion of a structural unit (b1) derived from the monomer (B1) in the copolymers is preferably from 0.1 to 64 mass%, more preferably from 1 to 60 mass%, still more preferably from 1 to 55 mass%, yet still more preferably from 5 to 50 mass%, yet still more preferably from 10 to 45 mass%, and particularly preferably from 20 to 45 mass%, per 100 mass% of the total amount of the structural units constituting the copolymer.

In the copolymer, the hydrophobic monomer (B) may contain a structural unit derived from a monomer having at least one functional group selected from the group consisting of a carboxyl group, a hydroxyl group, and an ether group, together with the structural unit derived from the (meth)acrylic acid alkyl ester. By copolymerizing the (meth)acrylic acid alkyl ester and the monomer having at least one functional group selected from the group consisting of a carboxyl group, a hydroxyl group, and an ether group as the hydrophobic monomer (B), the water solubility of the resulting copolymer is improved, and deposition of the copolymer due to a pH change can be more sufficiently suppressed. Therefore, the copolymer can be used in a wide pH range.

Examples of the monomer having at least one functional group selected from the group consisting of a carboxyl group, a hydroxyl group, and an ether group include the unsaturated monocarboxylic acid (salt) described above; a hydroxyl group-containing (meth)acrylate; an alkyl vinyl ether; an alkylene oxide adduct of an unsaturated alcohol having from 2 to 20 carbon atoms; and an addition reaction product of an unsaturated monomer having a cyclic ether-containing group having from 2 to 8 carbon atoms and an alcohol having from 1 to 20 carbon atoms.

Among them, the monomer having at least one functional group selected from the group consisting of a carboxyl group, a hydroxyl group, and an ether group is preferably an unsaturated monocarboxylic acid; a hydroxyl group-containing (meth)acrylate; an alkylene oxide adduct of an unsaturated alcohol having from 2 to 20 carbon atoms; or an addition reaction product of an unsaturated monomer having a cyclic ether-containing group having from 2 to 8 carbon atoms and an alcohol having from 1 to 20 carbon atoms. For the unsaturated monocarboxylic acid, (meth)acrylic acid and salts thereof are preferable. The hydroxyl group-containing (meth)acrylate is preferably 2-hydroxyethyl (meth)acrylate.

The number of carbon atoms of the unsaturated alcohol having from 2 to 20 carbon atoms is preferably from 2 to 18. Examples of the unsaturated alcohol having from 2 to 20 carbon atoms include vinyl alcohol, allyl alcohol, and isoprenyl alcohol. The number of carbon atoms of the alkylene oxide in the alkylene oxide adduct of an unsaturated alcohol having from 2 to 20 carbon atoms is preferably from 2 to 16, more preferably from 2 to 12, still more preferably from 2 to 6, yet still more preferably from 2 to 4, and particularly preferably from 2 to 3. Examples of the alkylene oxide include ethylene oxide, propylene oxide, butylene oxide, isobutylene oxide, 1-butene oxide, 2-butene oxide, and styrene oxide. Among them, ethylene oxide, propylene oxide, and butylene oxide are preferable, and ethylene oxide and propylene oxide are more preferable. An average number of moles of the alkylene oxide added is preferably from 1 to 100, more preferably from 1 to 80, still more preferably from 1 to 70, and particularly preferably from 1 to 50. The alkylene oxide adduct of an unsaturated alcohol having from 2 to 20 carbon atoms is preferably an ethylene oxide adduct of isoprenol.

Examples of the alcohol having from 1 to 20 carbon atoms include alkyl alcohols such as methanol, ethanol, propanol, butanol, pentanol, and hexanol. Among them, alkyl alcohols having from 2 to 16 carbon atoms such as ethanol, propanol, and butanol are preferable. As the addition reaction product of an unsaturated monomer having a cyclic ether-containing group having from 2 to 8 carbon atoms and an alcohol having from 1 to 20 carbon atoms, 1-allyloxy-3-butoxypropan-2-ol is preferable.

A content proportion of the structural unit (b) derived from the hydrophobic monomer (B) in the copolymers is preferably from 0.1 to 64 mass%, more preferably from 1 to 60 mass%, still more preferably from 2 to 55 mass%, yet still more preferably from 5 to 50 mass%, yet still more preferably from 10 to 45 mass%, and particularly preferably from 20 to 45 mass%, per 100 mass% of the total amount of the structural units constituting the copolymer.

The copolymer may have a structural unit (e) derived from an additional monomer (E) other than the primary to tertiary amino group-containing monomer (A) and the hydrophobic monomer (B). The additional monomer (E) is not particularly limited as long as it is a monomer copolymerizable with the primary to tertiary amino group-containing monomer (A) and/or the hydrophobic monomer (B). From the viewpoint of imparting antibacterial properties, the additional monomer (E) may contain a polymerizable metal salt. Examples of the polymerizable metal salt include heavy metal salts of unsaturated carboxylic acids such as zinc acrylate, zinc methacrylate, and zinc α-allyloxyacrylate.

In the copolymer, a content proportion of the structural unit (e) derived from the additional monomer (E) is preferably from 0 to 10 mass%, more preferably from 0 to 8 mass%, and still more preferably from 0 to 5 mass%, per 100 mass% of the total amount of the structural units constituting the copolymer.

A total content proportion of the structural unit (a) derived from the primary to tertiary amino group-containing monomer (A) and the structural unit (b) derived from the hydrophobic monomer (B) in the copolymer is preferably 60 mass% or more, more preferably 70 mass% or more, still more preferably 80 mass% or more, and particularly preferably 90 mass% or more, and may be 100 mass%, per 100 mass% of the total amount of the structural units constituting the copolymer.

From the viewpoint of adjusting the viscosity, the copolymer may contain a structural unit derived from a monomer having two or more ethylenically unsaturated groups, regardless of the value of the solubility parameter. Examples of the monomer having two or more ethylenically unsaturated groups include esters of polyols in which two or more hydroxyl groups are esterified with (meth)acrylic acid, examples of the polyols including ethylene glycol, propylene glycol, polyoxyethylene glycol, polyoxypropylene glycol, glycerin, polyglycerin, trimethylolpropane, pentaerythritol, saccharose, sorbitol, and 1,4-butanediol; ethers of the above-described polyols in which two or more hydroxyl groups are etherified with unsaturated alcohols such as allyl alcohol and vinyl alcohol; diallyl phthalate, triallyl phosphate, allyl methacrylate, tetraallyloxyethane, triallyl cyanurate, divinyl adipate, vinyl crotonate, 1,5-hexadiene, and divinylbenzene.

The copolymer may contain only one type of each of the monomers constituting the copolymer (the primary to tertiary amino group-containing monomer (A), the hydrophobic monomer (B), and the additional monomer (E)), or may contain two or more types of each of the monomers.

A weight average molecular weight (Mw) of the copolymer is preferably from 4000 to 1000000, more preferably from 4500 to 800000, still more preferably from 5000 to 600000, yet still more preferably from 6000 to 400000, yet still more preferably from 7000 to 200000, yet still more preferably from 10000 to 100000, and particularly preferably from 20000 to 80000. When the weight average molecular weight is within the above-described range, adsorptivity onto the skin or the like is improved, and thus the texture improving effect is more easily obtained. The weight average molecular weight can be measured by the method described in Examples.

The structure of the copolymer may be any structure including a random copolymer structure, a graft structure, a block copolymer structure, a gradient copolymer structure, a star structure, a dendrimer structure, and the like.

### Polyalkyleneimine

Examples of the polyalkyleneimine include a polymer of an alkyleneimine and a polymer of a polyamine. Examples of the alkyleneimine include alkyleneimines having from 2 to 6 carbon atoms such as ethyleneimine, propyleneimine, 1,2-butyleneimine, 2,3-butyleneimine, and 1,1-dimethylethyleneimine. Only one type of the above-described alkyleneimine may be used, or two or more types thereof may be used. That is, the polyalkyleneimine may be a homopolymer or a copolymer of alkyleneimine. Among them, the polyalkyleneimine is preferably a homopolymer of ethyleneimine (polyethyleneimine; PEI).

Examples of the polyamine include polyamines having from 2 to 6 carbon atoms such as ethylenediamine, diethylenetriamine, triethylenetetramine, and tetraethylenepentamine. Only one type of the above-described polyamine may be used, or two or more types thereof may be used. That is, the polyalkyleneimine may be a homopolymer or a copolymer of polyamine.

A weight average molecular weight (Mw) of the polyalkyleneimine is preferably 400 or more, more preferably 1000 or more, still more preferably 10000 or more, and yet still more preferably 50000 or more, from the viewpoint of safety to the skin. In addition, the weight average molecular weight is preferably 1000000 or less, more preferably 800000 or less, still more preferably 600000 or less, and particularly preferably 500000 or less from the viewpoint of more excellent feeling in use.

The weight average molecular weight can be measured by a gel permeation chromatography (GPC) method. Measurement conditions for the weight average molecular weight are preferably selected according to the weight average molecular weight of the polyalkyleneimine. For example, the measurement may be performed under the following Condition 1 when a low molecular weight polyalkyleneimine is measured, and the measurement may be performed under the following Condition 2 when a high molecular weight polyalkyleneimine is measured. For example, a molecular weight of about 100000 or less can be defined as a low molecular weight, and a molecular weight of more than about 100000 can be defined as a high molecular weight.

### Condition 1

### Measuring apparatus: available from Shimadzu Corporation

Column used: SHODEX Asahipac GF-710HQ + GF-510HQ + GF-310HQ, available from Showa Denko K.K.
Eluent: 0.2 mol%-monoethanolamine aqueous solution adjusted to pH 5.1 by adding acetic acid
Standard substance: Pullulan P-82 (available from FUJIFILM Wako Pure Chemical Corporation)
Detector: differential refractometer (available from Shimadzu Corporation)

### Condition 2

Measuring apparatus: available from Shimadzu Corporation
Column used: SHODEX OHpak SB-807HQ (two columns) + SB-806M/HQ (two columns) available from Showa Denko K.K.
Eluent: 0.5 mol%-sodium nitrate prepared to 0.5 mol%-acetic acid
Standard substance: Pullulan P-82 (available from FUJIFILM Wako Pure Chemical Corporation)
Detector: differential refractometer (available from Shimadzu Corporation)

An amine value per nonvolatile content of the polyalkyleneimine is preferably 5 or more, more preferably 10 or more, and still more preferably 15 or more. The amine value is preferably 30 or less, more preferably 25 or less, and still more preferably 20 or less.

The nonvolatile content (resin content) of the polyalkyleneimine can be measured by the Karl Fischer method or the dry weight method. The specific measurement method will be described below.

### · Karl Fischer method

Measuring instrument: Karl Fischer moisture meter
Solvent: from 20 to 30 ml of methanol
Amine neutralizing agent: 7 ml of acetic acid
Calculation formula: $resin content \left(wt.%\right) = 100 - V \times F / S \times 100$
V = KF titration amount (ml)
F = titer (mg/ml) of KF
S = amount (mg) of sample collected
· Dry weight method: About 1 g of a sample is collected in an aluminum dish, dried in a hot air circulation dryer at 150 ± 5°C for 1 hour, and then allowed to cool in a desiccator for 10 minutes.
Calculation formula: $resin content \left(wt.%\right) = W / S \times 100$
W = residual weight (g) after drying
S: sample weight (g) before drying

The amine value per nonvolatile content of the polyalkyleneimine is the number of moles (mmol) of amino groups contained in 1 g of the nonvolatile content of the polyalkyleneimine. The amine value of the polyethyleneimine can be calculated by potentiometric titration using a 0.5 mol/L p-toluenesulfonic acid standard solution in a methanolic solution.

A cationization degree of the polyalkyleneimine is preferably 5 meq/g or more, more preferably 10 meq/g or more, and still more preferably 15 meq/g or more. The cationization degree is preferably 30 meq/g or less, more preferably 25 meq/g or less, and still more preferably 22 meq/g or less.

The cationization degree can be measured by a known method, and can be determined from a measured value of an N content measured by, for example, the Kjeldahl method of Nitrogen Determination (Method II) in General Test Methods in the Standards of Cosmetic Ingredients, based on the following formula. The unit of cationization degree, meq/g, is the number of milliequivalents of cationic groups per g of polyalkyleneimine. 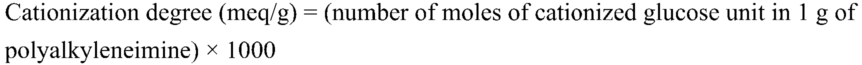 Cationization degree (meq/g) = (number of moles of cationized glucose unit in 1 g of polyalkyleneimine) × 1000 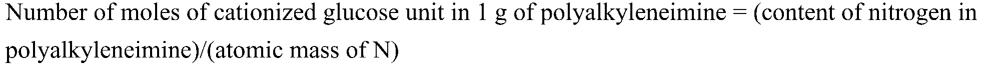 Number of moles of cationized glucose unit in 1 g of polyalkyleneimine = (content of nitrogen in polyalkyleneimine)/(atomic mass of N)

The polyalkyleneimine has at least one of a primary amine, a secondary amine, or a tertiary amine. A molar ratio of the primary amine, the secondary amine, and the tertiary amine in the polyalkyleneimine is, in terms of primary amine : secondary amine : tertiary amine, preferably from 10 to 50 : from 10 to 60 : from 10 to 50, more preferably from 20 to 45 : from 20 to 55 : from 10 to 40, and still more preferably from 25 to 40 : from 30 to 50 : from 20 to 35.

A content proportion of the primary amine in the polyalkyleneimine is preferably 10 mol% or more, more preferably 20 mol% or more, still more preferably 25 mol% or more, and particularly preferably 27 mol% or more, relative to a total amount (100 mol%) of the alkyleneimines constituting the polyalkyleneimine. The content proportion is also preferably 50 mol% or less, more preferably 45 mol% or less, still more preferably 40 mol% or less, and particularly preferably 37 mol% or less.

A content proportion of the secondary amine in the polyalkyleneimine is preferably 10 mol% or more, more preferably 20 mol% or more, and still more preferably 30 mol% or more, relative to the total amount (100 mol%) of the alkyleneimines constituting the polyalkyleneimine. The content proportion is also preferably 60 mol% or less, more preferably 55 mol% or less, and still more preferably 50 mol% or less.

A content proportion of the tertiary amine in the polyalkyleneimine is preferably 10 mol% or more, more preferably 15 mol% or more, and still more preferably 20 mol% or more, relative to the total amount (100 mol%) of the alkyleneimines constituting the polyalkyleneimine. The content proportion is also preferably 50 mol% or less, more preferably 40 mol% or less, and still more preferably 35 mol% or less.

The polyalkyleneimine may be a modified polyalkyleneimine (modified polyalkyleneimine). Examples of the modified polyalkyleneimine include (i) a compound obtained by adding, for example, from 1 mol to 300 mol of an alkylene oxide having from 2 to 30 carbon atoms and/or a glycidyl ether compound to at least a part of the primary amino group and/or the secondary amino group contained in the polyalkyleneimine relative to 1 mol of active hydrogen of the amino groups, (ii) a compound obtained by Michael addition of a compound having a carbon-carbon unsaturated double bond such as acrylic acid, an acrylic acid ester, styrene, acrylonitrile, N-vinylpyrrolidone, or vinyl acetate to at least a part of the primary amino group and/or the secondary amino group contained in the polyalkyleneimine, and (iii) a compound obtained by addition of an isocyanate group-containing compound, an ester group-containing compound, a ketone group-containing compound, an acid anhydride, or the like to at least a part of the primary amino group and/or the secondary amino group contained in the polyalkyleneimine.

When the polyalkyleneimine contains a modified polyalkyleneimine to which an alkylene oxide is added, an amount of the alkylene oxide added is preferably 10 parts by mass or less per 100 parts by mass of the polyalkyleneimine from the viewpoint of reducing an unpleasant odor. The amount of the alkylene oxide added is preferably 5 parts by mass or less, and more preferably 3 parts by mass or less.

The polyalkyleneimine and/or the modified polyalkyleneimine (modified polyalkyleneimine) may be a neutralized product obtained by neutralizing the primary to tertiary amino group with an acid. Examples of a counter anion of the acid include those exemplified and described as Y⁻ in Formula (1').

The polyalkyleneimine may be linear or may have a branched structure. A degree of branching of the polyalkyleneimine is preferably more than 0%, more preferably 1% or more, still more preferably 5% or more, yet still more preferably 10% or more, and particularly preferably 15% or more. The degree of branching is preferably 50% or less, more preferably 40% or less, and still more preferably 35% or less.

The degree of branching of the polyalkyleneimine can be calculated from the following formula from the number a of tertiary amines and the number b of secondary amines, by determining an intensity ratio of the carbon atoms bonded to the tertiary amines and the carbon atoms bonded to the secondary amines from a chart obtained by measuring ¹³C-NMR of the polyethyleneimine. That is, the linear polyethyleneimine has no tertiary amine, and therefore the degree of branching is 0%. A polyethylene in which all nitrogen atoms are tertiary amines, i.e., maximally branched, has a degree of branching of 100%. $Degree of branching \left(%\right) = \left[a/\left(a+b\right)\right] \times 100$

### Composition

The composition of the present invention is a composition containing the primary to tertiary amino group-containing polymer compound and an inorganic powder. In the composition, the texture improver may be used together with the primary to tertiary amino group-containing polymer compound or instead of the primary to tertiary amino group-containing polymer compound. That is, the composition may be a composition containing the texture improver and an inorganic powder. The composition exhibits effects corresponding to the inorganic powder to be blended, and is excellent in dispersibility of the inorganic powder. This improves the texture of the composition as compared with when only an inorganic powder is blended. Only one type of each of the primary to tertiary amino group-containing polymer compound and the texture improver may be used, or two or more types thereof may be used.

As the inorganic powder, for example, known or commonly used inorganic powders used in cosmetic compositions can be used. Examples of the inorganic powder include metal oxides such as titanium oxide, red iron oxide, yellow iron oxide, black iron oxide, zinc oxide, cerium oxide, chromium oxide, chromium hydroxide, magnesium oxide, and zirconium oxide; and inorganic pigments such as manganese violet, cobalt violet, cobalt titanate, ultramarine, Prussian blue, talc, kaolin, sericite, muscovite, phlogopite, synthetic mica, lepidolite, biotite, synthetic phlogopite (synthetic fluorophlogopite), and amino acid-based powders (lauroyllysine), permecurite, silica, calcium carbonate, magnesium carbonate, magnesium silicate, aluminum silicate, barium silicate, calcium silicate, barium sulfate, calcium sulfate, calcium phosphate, hydroxyapatite, boron nitride, pearl pigments, and bismuth oxychloride. Only one type of the above-described inorganic powder may be used, or two or more types thereof may be used.

The surface of the inorganic powder may be hydrophilic or hydrophobic. In any case, the dispersibility of the inorganic powder in the composition is excellent by blending the inorganic powder together with the primary to tertiary amino group-containing polymer compound. In particular, when the surface is hydrophobic, the dispersibility improving effect and the texture improving effect are remarkably obtained.

Examples of the inorganic powder having a hydrophobic surface include inorganic powders subjected to a hydrophobic treatment. Examples of a surface treatment agent that performs the surface treatment include metal oxides, silane coupling agents, titanium coupling agents, organic acids, polyols, and organosilicon compounds. Examples of the metal oxide include aluminum hydroxide. Examples of the silane coupling agent include β-(3,4-epoxycyclohexyl)ethyltrimethoxysilane, γ-glycidoxypropyltrimethoxysilane, and γ-glycidoxypropylmethyldiethoxysilane. Examples of the organic acid include stearic acid. Examples of the organosilicon compound include trimethylchlorosilane, hexamethyldisiloxane, dimethyldichlorosilane, octamethylcyclotetrasilane, polydimethylsiloxane, hexadecylsilane, methacrylsilane, and silicone oil. Only one type of the surface treatment agent may be used, or two or more types thereof may be used.

The composition may be in a liquid form or in a solid form. Examples of the composition in a liquid form include a composition containing a solvent such as water and having the primary to tertiary amino group-containing polymer compound and the inorganic powder dispersed in the solvent. Examples of the composition in a solid form include a solvent-free composition containing the primary to tertiary amino group-containing polymer compound and the inorganic powder. The composition in a solid form provides excellent compatibility and feeling of adhesion of the inorganic powder with/to the skin, and also has an effect that the inorganic powder is less likely to fall off after application to the skin. The composition in a liquid form is excellent in dispersibility of the inorganic powder in the composition, and excellent in compatibility of the inorganic powder with the skin and moist feeling.

The content proportion of the inorganic powder in the composition is, for example, from 0.5 to 99.99 mass%, preferably from 1 to 99.97 mass%, and more preferably from 5 to 99.95 mass%, per 100 mass% of the total amount of the composition. When the content proportion is within the above-described range, the texture when the composition is used is more favorable.

In one embodiment, the content proportion of the inorganic powder in the composition is, for example, from 0.5 to 60 mass%, preferably from 1 to 40 mass%, and more preferably from 5 to 20 mass%, per 100 mass% of the total amount of the composition. When the content proportion is within the above-described range, the dispersibility of the inorganic powder is more favorable when the composition is used in a liquid form.

In another embodiment, the content proportion of the inorganic powder in the composition is, for example, from 70 to 99.99 mass%, preferably from 80 to 99.97 mass%, and more preferably from 90 to 99.95 mass%, per 100 mass% of the total amount of the composition. When the content proportion is within the above-described range, it is expected that the texture (particularly, adhesion to the skin and suppression of stickiness) when the composition is used as a solid (for example, powder) is favorable.

A content of the primary to tertiary amino group-containing polymer compound in the composition is preferably from 0.01 to 300 parts by mass, more preferably from 0.03 to 250 parts by mass, still more preferably from 0.05 to 200 parts by mass, and particularly preferably from 0.07 to 150 parts by mass, per 100 parts by mass of a total amount of the inorganic powder. When the content is within the above-described range, the texture improving effect of the inorganic powder is more favorable.

In one embodiment, a content of the primary to tertiary amino group-containing polymer compound in the composition is preferably from 0.5 to 300 parts by mass, more preferably from 1 to 250 parts by mass, still more preferably from 2 to 200 parts by mass, and particularly preferably from 3 to 150 parts by mass, per 100 parts by mass of the total amount of the inorganic powder. When the content is within the above-described range, the dispersibility of the inorganic powder and the texture improving effect are more favorable.

In another embodiment, the content of the primary to tertiary amino group-containing polymer compound in the composition is, for example, from 0.01 to 200 parts by mass, preferably from 0.03 to 100 parts by mass, more preferably from 0.05 to 50 parts by mass, still more preferably from 0.07 to 15 parts by mass, and particularly preferably from 0.08 to 5 parts by mass, per 1000 parts by mass of the total amount of the inorganic powder. When the content proportion is within the above-described range, it is expected that the texture (particularly, adhesion to the skin and suppression of stickiness) when the composition is used as a solid (for example, powder) is favorable.

In the present invention, the composition containing the primary to tertiary amino group-containing polymer compound and the inorganic powder may be in an aspect in which the primary to tertiary amino group-containing polymer compound and the inorganic powder are separately present in the composition, or may be in an aspect in which the primary to tertiary amino group-containing polymer compound is present on the surface of the inorganic powder, but is preferably in an aspect in which the primary to tertiary amino group-containing polymer compound is present on the surface of the inorganic powder from the viewpoint of the texture improving properties when the composition is used by being blended in a cosmetic.

In the present invention, the composition containing the texture improver and the inorganic powder may be in an aspect in which the texture improver and the inorganic powder are present separately in the composition, or may be in an aspect in which the texture improver is present on the surface of the inorganic powder, but is preferably in an aspect in which the texture improver is present on the surface of the inorganic powder from the viewpoint of the texture improving properties when the composition is used by being blended in a cosmetic.

As a means that confirms that the texture improver is present on the surface of the inorganic powder, a known means can be used. For example, it can be confirmed whether or not an organic substance used in the texture improver is present on the surface of the inorganic powder by using both methods of an X-ray diffraction method (XRD) and organic element analysis.

When the X-ray diffraction method and the organic element analysis value are used as the means that confirms that the texture improver is present on the surface of the inorganic powder, it is possible to determine whether the inorganic powder has the texture improver present on the surface thereof by confirming an element used in the texture improver such as nitrogen, oxygen, or carbon with respect to the powder confirmed to be a specific inorganic powder from the diffraction pattern.

The inorganic powder having the texture improver present on the surface thereof may be dispersed in a solvent, or may be obtained as a powder from which the solvent has been removed. In the composition, the texture improver and the inorganic powder may be dispersed in a solvent, or may be obtained as a powder from which the solvent has been removed. The inorganic powder having the texture improver present on the surface thereof is obtained through a step of mixing the texture improver and the inorganic powder in a solvent (mixing step). For the inorganic powder having the texture improver present on the surface thereof, in order to obtain a powder from which the solvent has been removed, the inorganic powder preferably undergoes a step of removing the solvent.

The step of removing the solvent may include a concentration step, a filtration step, a classification step, a drying step, and the like, and more preferably includes a drying step.

The drying step may be selected from heating drying, drying under reduced pressure, heating drying under reduced pressure, freeze drying, and the like, and as an apparatus used in the drying step, a batch-type drying apparatus such as a vacuum box dryer, a through-flow dryer, a rotary evaporator, or a stirring dryer, a spray drying type dryer, a screw conveyor type dryer, or a drum type dryer may be used.

In order to further improve the texture of the inorganic powder having the texture improver present on the surface thereof, the method may include pulverizing the inorganic powder (pulverization step). The pulverization step is preferably performed using a pulverizer. The pulverizer is not particularly limited, and examples thereof include a roll type pulverizer such as a roll mill, a hammer type pulverizer such as a hammer mill, an impact type pulverizer, a cutter mill, a turbo grinder, a ball mill, a pin mill, a flash mill, and a jet mill such as a fluidized bed type jet mill or a target type jet mill. A high-speed fluidizing type mixer can also be used as the pulverizer. A pulverization method in the pulverization step is not particularly limited, and pulverization may be performed at room temperature or may be performed by freeze pulverization.

A zeta potential of the composition is preferably positive, more preferably 1 mV or higher, still more preferably 5 mV or higher, yet still more preferably 10 mV or higher, and particularly preferably 20 mV or higher. When the zeta potential is positive, it is presumed that cationization of the surface of the inorganic powder has proceeded by the primary to tertiary amino group-containing polymer compound, and the dispersibility of the inorganic powder and the texture improving effect are more favorable. The zeta potential of the composition may be negative. In this case, the zeta potential is preferably -5 mV or lower, and more preferably -10 mV or lower, and may be -20 mV or lower or -30 mV or lower. The zeta potential is, for example, -40 mV or higher. Even when the zeta potential is negative, the dispersibility of the inorganic powder and the texture improving effect are more favorable. The zeta potential when the composition is in a liquid form (slurry form) is a value when the composition is dispersed in water so that a concentration of the inorganic powder is 0.03 mass%, and the zeta potential when the composition is in a solid form (for example, powder) is a value when the composition is dispersed in water so that the concentration of the composition is 0.03 mass%. In the zeta potential measurement, the liquid form means a form having fluidity at normal temperature (25°C) and normal pressure (including a form having a state of being dispersed in a solvent), and the solid form means a semi-solid to solid state having no fluidity at normal temperature (25°C) and normal pressure (including a powder state).

A solids content concentration of the composition is, for example, from 1 to 50 mass%, preferably from 5 to 30 mass%, and more preferably from 7 to 20 mass%, per 100 mass% of the total amount of the composition. When the solids content concentration is within the above-described range, the dispersibility of the inorganic powder in the composition in a liquid form and the texture improving effect are more favorable. In the composition in a solid form, the solids content concentration of the composition may be 90 mass% or more, 95 mass% or more, 98 mass% or more, 99 mass% or more, or 95.5 mass% or more, per 100 mass% of the total amount of the composition.

### Additional components

The composition may contain an additional component other than the above-described components. Examples of the additional component include solvents (for example, water and organic solvents), oil agents, lower alcohols, polyhydric alcohols, thickeners, humectants, surfactants (anionic surfactants, cationic surfactants, amphoteric surfactants, and nonionic surfactants), fatty acid alkanolamides, antioxidants, antioxidation aids, powder components (for example, organic powders, pigments, and dyes), natural water-soluble polymers, semisynthetic water-soluble polymers, synthetic water-soluble polymers, chelating agents, sugars and derivatives thereof, amino acids and derivatives thereof, organic amines, polymer emulsions, pH adjusters (acids, alkalis, and the like), vitamins, preservatives/antimicrobial agents, antiphlogistics, various extracts, activators, blood circulation promoters, antiseborrheic agents, anti-inflammatory agents, and perfumes. Only one type of the above-described additional component may be used, or two or more types thereof may be used.

A content proportion of the solvent (particularly, water) in the composition in a liquid form is preferably 60 mass% or more, more preferably 70 mass% or more, and particularly preferably 80 mass% or more, per 100 mass% of the total amount of the composition.

The composition can be produced by a known or commonly used method. For example, the composition can be produced by mixing and stirring the above-described components.

### Cosmetic

The composition can be used to make a cosmetic. The cosmetic containing the composition is sometimes referred to as "the cosmetic of the present invention". That is, the composition is preferably a cosmetic composition. The cosmetic of the present invention contains the composition, and thus is excellent in dispersibility of the inorganic powder while exhibiting effects corresponding to the inorganic powder to be blended. In addition, the texture is improved as compared with the case where only the inorganic powder is blended.

The cosmetic of the present invention may contain an additional component other than the composition. The cosmetic of the present invention may be the composition of the present invention itself, or may be produced by adding various components to the composition of the present invention, for example. Examples of the various components include those exemplified and described as additional components that may be contained in the composition of the present invention. Only one type of the above-described various components may be used, or two or more types thereof may be used.

A content proportion of the water in the cosmetic amount is preferably 60 mass% or more, more preferably 70 mass% or more, and particularly preferably 80 mass% or more, per 100 mass% of the total amount of the composition.

Examples of the cosmetic include hair cosmetics such as shampoos, rinses (hair conditioners), hair treatments, hair foams, hair gels, hair waters, hair waxes, hair creams, hair colors, hair bleaches, and permanent wave agents; and skin cosmetics such as creams, facial cleansing foams, liquid soaps, hand soaps, body soaps, liquid cleaners for babies, shampoos for pets, cleansing creams, cleansing milks, cleansing lotions, bath agents for foam baths, antiperspirants, massage creams, moisturizing creams, sunscreen creams, hand creams, body shampoos, lipsticks, liquid foundations, skin lotions, cosmetic liquids, milky lotions, colognes, and nail cosmetics. In particular, from the viewpoint of an excellent texture improving effect, a skin cosmetic is preferable.

### Examples

The present invention will be described in more detail below by way of Examples, but is not limited to these Examples. Note that "parts" represents "parts by mass" and "%" represents "mass%" unless otherwise specified.

### Production Example 1

A glass separable flask equipped with a thermometer, a reflux condenser, and a stirrer was charged with 11.1 g of pure water and 100.0 g of 1,3-butanediol (available from Daicel Corporation), and the temperature was raised to 90°C under stirring. Subsequently, a monomer solution 1 containing 60.0 g of 2-(dimethylamino)ethyl methacrylate (also known as N,N-dimethylaminoethyl methacrylate) (available from Kyoeisha Chemical Co., Ltd.), a monomer solution 2 containing 40.0 g of ethyl methacrylate (available from Kyoeisha Chemical Co., Ltd.), and an aqueous initiator solution containing 36.6 g of a 3% aqueous 2,2'-azobis(2-methylpropionamidine) dihydrochloride (available from FUJIFILM Wako Pure Chemical Corporation) solution were added dropwise to a polymerization reaction system in a constant state at 90°C from separate dropwise addition nozzles with stirring. With respect to the dropwise addition start time, the monomer solutions 1 and 2 and the aqueous initiator solution were added dropwise simultaneously, and the monomer solution 1 was added dropwise for 180 minutes, the monomer solution 2 was added dropwise for 170 minutes, and the aqueous initiator solution was added dropwise for 210 minutes. After completion of the entire dropwise addition, the reaction solution was further kept at 90°C for 30 minutes and aged to complete the polymerization, and then 200 g of pure water and 552 g of 1,3-butanediol were added to obtain a polymer solution containing a copolymer. The obtained copolymer had a solids content of 9.9%, a pH of 9.0, and a weight average molecular weight of 48000.

### Method for Measuring Weight Average Molecular Weight

The weight average molecular weight (Mw) of the copolymer was measured by gel permeation chromatography (GPC). The measurement conditions, apparatuses, and the like were as follows.
Apparatus: e2695 available from Waters Corporation
Detector: differential refractive index (RI) detector
Column: TSKgel α-M, α-2500 available from Tosoh Corporation
Column temperature: 40°C
Flow rate: 0.8 mL/min
Injection volume: 10 µL (eluent preparation solution with a sample concentration of 0.4 wt.%)
Calibration curve: polyethylene glycol available from GL Sciences Inc.
GPC software: EMPOWER3 available from Waters Corporation
Eluent: 0.5 M acetic acid + 0.2 M Na nitrate/acetonitrile = 50/50 (v/v)

### Method for Measuring Solids Content

About 1 g of the obtained polymer solution was weighed, and dried in a hot air dryer at 200°C for 15 minutes. Then, the mass of the residue was taken as the solids content, and the ratio of the solids content to the mass before drying was expressed in %.

### pH Measurement

The value of the obtained polymer solution at 25°C was measured using a pH meter ("F-72" available from HORIBA, Ltd.).

### Example 1

A glass screw tube was charged with 1.0 g of titanium oxide (trade name: SP Solaveil XTP1 MBAL, available from Croda Japan K. K.) and 10.0 g of a 10% aqueous polyethyleneimine (weight average molecular weight: 350000, available from Nippon Shokubai Co., Ltd.) solution, and they were stirred with a stirring rod for 5 minutes to obtain a slurry liquid.

### Example 2

A glass screw tube was charged with 1.0 g of titanium oxide (trade name: SP Solaveil XTP1 MBAL, available from Croda Japan K. K.) and 10.0 g of the polymer solution obtained in Production Example 1, and they were stirred with a stirring rod for 5 minutes to obtain a slurry liquid.

### Example 3

A glass screw tube was charged with 1.0 g of titanium oxide (trade name: MT-100TV, available from Tayca Corporation) and 10.0 g of a 10% aqueous polyethyleneimine (weight average molecular weight: 350000, available from Nippon Shokubai Co., Ltd.) solution, and they were stirred with a stirring rod for 5 minutes to obtain a slurry liquid.

### Example 4

A glass screw tube was charged with 1.0 g of titanium oxide (trade name: MT-100TV, available from Tayca Corporation) and 10.0 g of the polymer solution obtained in Production Example 1, and they were stirred with a stirring rod for 5 minutes to obtain a slurry liquid.

### Comparative Example 1

A glass screw tube was charged with 1.0 g of titanium oxide (trade name: SP Solaveil XTP1 MBAL, available from Croda Japan K. K.) and 10.0 g of ion-exchanged water, and they were stirred with a stirring rod for 5 minutes to obtain a slurry liquid.

### Comparative Example 2

A glass screw tube was charged with 1.0 g of titanium oxide (trade name: MT-100TV, available from Tayca Corporation) and 10.0 g of ion-exchanged water, and they were stirred with a stirring rod for 5 minutes to obtain a slurry liquid.

### Evaluation

The slurry liquids obtained in the examples and comparative examples were evaluated as follows. The results are presented in Table 3.

### (1) Zeta Potential

The slurry liquids obtained in Examples 1 to 4 and Comparative Examples 1 and 2 were diluted with ion-exchanged water so that the concentration of titanium oxide was 0.03%, and then the zeta potential was measured using a zeta potential measuring apparatus (Zetasizer Ultra available from Malvern Panalytical).

### (2) Dispersibility

The appearance of the slurry solution obtained in each of the examples and comparative examples was observed 10 minutes and 24 hours after the stirring was stopped, and the "dispersibility" was evaluated according to the criteria presented in Table 1.

### [Table 1]

**Table 1**

| Dispersibility | Evaluation criteria |
|---|---|
| ⊚ | The inorganic powder is uniformly dispersed in the solution |
| ○ | A part of the inorganic powder is precipitated in the solution |
| Δ | The majority of the inorganic powder is precipitated in the solution |
| × | The inorganic powder floats in a state of being separated on the liquid surface |

### (3) Texture

The slurry solution obtained in each of the examples and comparative examples was rubbed on the palm of the hand, and the "texture" was evaluated by five expert panelists according to the criteria presented in Table 2. From the evaluation results of the five panelists, the determination result selected by the largest number of panelists is presented in Table 3.

### [Table 2]

**Table 2**

| Texture | Evaluation criteria |
|---|---|
| ⊚ | When rubbed, the slurry solution is compatible with the skin and gives a moist texture |
| × | When rubbed, the slurry solution gives a powdery texture |

### [Table 3]

**Table 3**

| | Example 1 | Example 2 | Example 3 | Example 4 | Comparative Example 1 | Comparative Example 2 |
|---|---|---|---|---|---|---|
| Zeta potential [mV] | 27.9 | -17.2 | 25.7 | -11.2 | -8.9 | -29.0 |
| Dispersibility (after 10 minutes) | ⊚ | ⊚ | ⊚ | ⊚ | × | × |
| Dispersibility (after 24 hours) | ○ | ○ | ⊚ | ⊚ | × | × |
| Texture | ⊚ | ⊚ | ⊚ | ⊚ | × | × |

As presented in Table 3, the slurry liquids of Examples 1 to 4 were excellent in dispersibility of titanium oxide as compared with the comparative examples not containing the specific polymer compound. In addition, it was confirmed that the texture was improved, functioning as texture improvers.

### Example 5

A glass screw tube was charged with 1.0 g of titanium oxide (trade name: MP-1133, available from Tayca Corporation) and 10.0 g of a 1% aqueous polyethyleneimine (weight average molecular weight: 350000, available from Nippon Shokubai Co., Ltd.) solution, and they were stirred with a stirring rod for 5 minutes to obtain a slurry liquid. The obtained slurry liquid was dried for 2 days by a freeze dryer ("FDU-2200" available from Tokyo Rikakikai Co., Ltd.) to obtain a dry powder.

### Example 6

A glass screw tube was charged with 1.0 g of titanium oxide (trade name: MP-1133, available from Tayca Corporation) and 10.0 g of a 0.1% aqueous polyethyleneimine (weight average molecular weight: 350000, available from Nippon Shokubai Co., Ltd.) solution, and they were stirred with a stirring rod for 5 minutes to obtain a slurry liquid. The obtained slurry liquid was dried for 2 days by a freeze dryer ("FDU-2200" available from Tokyo Rikakikai Co., Ltd.) to obtain a dry powder.

### Example 7

A glass screw tube was charged with 1.0 g of titanium oxide (trade name: MP-1133, available from Tayca Corporation) and 10.0 g of a 0.01% aqueous polyethyleneimine (weight average molecular weight: 350000, available from Nippon Shokubai Co., Ltd.) solution, and they were stirred with a stirring rod for 5 minutes to obtain a slurry liquid. The obtained slurry liquid was dried for 2 days by a freeze dryer ("FDU-2200" available from Tokyo Rikakikai Co., Ltd.) to obtain a dry powder.

### Example 8

A glass screw tube was charged with 1.0 g of talc (trade name: JA-46R, available from Asada Milling Co., Ltd.) and 10.0 g of a 1% aqueous polyethyleneimine (weight average molecular weight: 350000, available from Nippon Shokubai Co., Ltd.) solution, and they were stirred with a stirring rod for 5 minutes to obtain a slurry liquid. The obtained slurry liquid was dried for 2 days by a freeze dryer ("FDU-2200" available from Tokyo Rikakikai Co., Ltd.) to obtain a dry powder.

### Example 9

A glass screw tube was charged with 1.0 g of talc (trade name: JA-46R, available from Asada Milling Co., Ltd.) and 10.0 g of a 0.1% aqueous polyethyleneimine (weight average molecular weight: 350000, available from Nippon Shokubai Co., Ltd.) solution, and they were stirred with a stirring rod for 5 minutes to obtain a slurry liquid. The obtained slurry liquid was dried for 2 days by a freeze dryer ("FDU-2200" available from Tokyo Rikakikai Co., Ltd.) to obtain a dry powder.

### Example 10

A glass screw tube was charged with 1.0 g of talc (trade name: JA-46R, available from Asada Milling Co., Ltd.) and 10.0 g of a 0.01% aqueous polyethyleneimine (weight average molecular weight: 350000, available from Nippon Shokubai Co., Ltd.) solution, and they were stirred with a stirring rod for 5 minutes to obtain a slurry liquid. The obtained slurry liquid was dried for 2 days by a freeze dryer ("FDU-2200" available from Tokyo Rikakikai Co., Ltd.) to obtain a dry powder.

### Comparative Example 3

A glass screw tube was charged with 1.0 g of titanium oxide (trade name: MP-1133, available from Tayca Corporation) and 10.0 g of ion-exchanged water, and they were stirred with a stirring rod for 5 minutes to obtain a slurry liquid. The obtained slurry liquid was dried for 2 days by a freeze dryer ("FDU-2200" available from Tokyo Rikakikai Co., Ltd.) to obtain a dry powder.

### Comparative Example 4

A glass screw tube was charged with 1.0 g of talc (trade name: JA-46R, available from Asada Milling Co., Ltd.) and 10.0 g of ion-exchanged water, and they were stirred with a stirring rod for 5 minutes to obtain a slurry liquid. The obtained slurry liquid was dried for 2 days by a freeze dryer ("FDU-2200" available from Tokyo Rikakikai Co., Ltd.) to obtain a dry powder.

The information on the titanium oxide and talc used in the examples and comparative examples is presented below.

### -Titanium Oxide-

SP Solaveil XTP1 MBAL: hydrophobic titanium oxide (alumina and stearic acid-treated titanium oxide), average particle size: 179 nm
MT-100TV: hydrophobic titanium oxide (aluminum hydroxide and stearic acid-treated titanium oxide), average particle size: 15 nm
MP-1133: hydrophilic titanium oxide (aluminum hydroxide-treated titanium oxide), average particle size: 250 nm

### -Talc-

JA-46R: hydrophilic talc, average particle size: from 7.0 to 11.0 µm

### Evaluation

The powders obtained in the examples and comparative examples were evaluated as follows. The results are presented in Table 5.

### (1) Zeta Potential

The powders obtained in Examples 5 to 10 and Comparative Examples 3 and 4 were diluted with ion-exchanged water so that the concentration of the powder was 0.03%, and then the zeta potential was measured using a zeta potential measuring apparatus (Zetasizer Ultra available from Malvern Panalytical).

### (2) Dispersibility

The powder obtained in each of the examples and comparative examples (1.0 g) and ion-exchanged water (10.0 g) were charged and stirred with a stirring rod for 5 minutes. The appearance was observed 10 minutes and 24 hours after the stirring was stopped, and the "dispersibility" was evaluated according to the criteria presented in Table 1.

### (3) Texture

The powder obtained in each of the examples and comparative examples was rubbed on the palm of the hand, and the "texture" was evaluated by five expert panelists according to the criteria presented in Table 4. From the evaluation results of the five panelists, the determination result selected by the largest number of panelists is presented in Table 5.

### [Table 4]

**Table 4**

| Texture | Evaluation criteria |
|---|---|
| ⊚ | When rubbed, the powder is compatible with the skin and gives a feeling of adhesion, |
| | and |
| | when rubbed again, the powder does not fall off the skin |
| ○ | When rubbed, the powder is compatible with the skin and gives a feeling of adhesion, |
| | but |
| | when rubbed again, the powder slightly falls off the skin |
| × | When rubbed, the powder does not give a feeling of adhesion, and when rubbed again, the powder falls off the skin |

### [Table 5]

**Table 5**

| | Example 5 | Example 6 | Example 7 | EXAMPLE 8 | Example 9 | Example 10 | Comparative Example 3 | Comparative Example 4 |
|---|---|---|---|---|---|---|---|---|
| Zeta potential [mV] | 70.1 | 68.3 | 56.9 | 68.9 | 61.7 | 70.4 | 54.0 | -26.6 |
| Dispersibility (after 10 minutes) | ⊚ | ⊚ | ⊚ | ⊚ | ⊚ | ⊚ | × | × |
| Dispersibility (after 24 hours) | ○ | ○ | ○ | ○ | ○ | ○ | × | × |
| Texture | ○ | ⊚ | ⊚ | ○ | ⊚ | ⊚ | × | × |

As presented in Table 5, the powders of Examples 5 to 10 were excellent in dispersibility of titanium oxide and talc as compared with the comparative examples not containing the specific polymer compound. In addition, it was confirmed that the texture was improved, functioning as texture improvers.

### Example 11

A 5-liter Henschel mixer (product name: FM5, available from Nippon Coke & Engineering Co., Ltd.) was charged with 1.0 kg of talc (trade name: JA-46R, available from Asada Milling Co., Ltd.), and a liquid obtained by mixing 33.33 g of a 30% aqueous polyethyleneimine (weight average molecular weight: 350000, available from Nippon Shokubai Co., Ltd.) solution and 166.67 g of ethanol was added dropwise thereto under stirring with N₂ gas as seal air. After the dropwise addition, the mixture was heated under stirring and mixed under heating at 60°C for 1 hour to obtain a dry powder.

### Comparative Example 5

Surface-uncoated talc (trade name: JA-46R, available from Asada Milling Co., Ltd.) was used as a powder.

The information on the talc used in the examples and comparative examples is presented below.

### -Talc-

JA-46R: hydrophilic talc, average particle size: from 7.0 to 11.0 µm

### Evaluation

The powders obtained in the examples and comparative examples were evaluated as follows. The results are presented in Table 7 and FIG. 1.

### (1) Zeta Potential

The powder obtained in Example 11 and the powder of Comparative Example 5 were diluted with ion-exchanged water so that the concentration of the powder was 0.03%, and then the zeta potential was measured using a zeta potential measuring apparatus (Zetasizer Ultra available from Malvern Panalytical).

### (2) Organic Element Analysis Measurement (Nitrogen Content Measurement)

Contents of nitrogen contained in the powder obtained in Example 11 and the powder of Comparative Example 5 were measured using an organic element analyzer (Vario EL cube available from Elementar).

### (3) Moisture Amount

The powder obtained in Example 11 and the powder of Comparative Example 5 (1.0 g) were weighed, and then dried at 120°C for 4 hours using a hot air dryer (SI401, available from Yamato Scientific Co., Ltd.). After drying, the weight was measured with a precision balance, and the weight loss on drying was calculated.

### (4) Dispersibility

The powder obtained in Example 11 and the powder of Comparative Example 5 (1.0 g) and ion-exchanged water (10.0 g) were charged and stirred with a stirring rod for 5 minutes. The appearance was observed 10 minutes and 24 hours after the stirring was stopped, and the "dispersibility" was evaluated according to the criteria presented in Table 1.

### (5) Texture

The powder obtained in Example 11 and the powder of Comparative Example 5 were each rubbed on the palm of the hand, and the "texture" was evaluated by five expert panelists according to the criteria presented in Table 6. From the evaluation results of the five panelists, the determination result selected by the largest number of panelists is presented in Table 7.

### [Table 6]

**Table 6**

| Texture | Evaluation criteria |
|---|---|
| ⊚ | When rubbed, the powder gives a moist feeling and a feeling of adhesion, and when rubbed again, the powder does not fall off the skin |
| × | When rubbed, the powder gives neither a moist feeling nor a feeling of adhesion, and when rubbed again, the powder falls off the skin |

### (6) Particle Size Distribution

The particle size distributions of the powder obtained in Example 11 and the powder of Comparative Example 5 were measured using a laser diffraction type particle size distribution measuring apparatus (Mastersizer 3000 + available from Malvern Panalytical). The results are presented in FIG. 1.

### [Table 7]

**Table 7**

| | Example 11 | Comparative Example 5 |
|---|---|---|
| Zeta potential [mV] | 56.9 | -20.0 |
| Nitrogen content [%] | 0.32 | 0.00 |
| Moisture amount [%] | 0.49 | 0.13 |
| Dispersibility (after 10 minutes) | ⊚ | × |
| Dispersibility (after 24 hours) | ○ | × |
| Texture | ⊚ | × |

As presented in Table 7 and FIG. 1, the powder of Example 11 had a sharp particle size distribution, an increased moisture amount, improved talc dispersibility, and increased zeta potential and nitrogen content, as compared with Comparative Example 5, which is surface-uncoated talc. Therefore, it was confirmed that polyethyleneimine was present on the surface of the powder of Example 11. Further, it was confirmed that such a powder having polyethyleneimine present on a surface thereof has an improved texture and functions as a texture improver.

Hereinafter, variations of the invention according to the present invention will be described.

[Appendix 1] A texture improver, containing a primary to tertiary amino group-containing polymer compound.

[Appendix 2] The texture improver according to Appendix 1, wherein the primary to tertiary amino group-containing polymer compound includes: a copolymer containing a structural unit (a) derived from a primary to tertiary amino group-containing monomer (A) and a structural unit (b) derived from a hydrophobic monomer (B); and/or a polyalkyleneimine.

[Appendix 3] The texture improver according to Appendix 2, wherein the primary to tertiary amino group-containing monomer (A) is preferably an N,N-dialkylamino group-containing (meth)acrylate or an N,N-dialkylamino group-containing (meth)acrylamide, more preferably one or more selected from the group consisting of N,N-dimethylaminoethyl (meth)acrylate, N,N-diethylaminoethyl (meth)acrylate, and N,N-dimethylaminopropyl (meth)acrylamide.

[Appendix 4] The texture improver according to Appendix 2 or 3, wherein a content proportion of the structural unit (a) derived from the primary to tertiary amino group-containing monomer (A) in the copolymer is from 36 to 99.9 mass% (preferably from 40 to 99 mass%, more preferably from 45 to 98 mass%, still more preferably from 50 to 95 mass%, yet still more preferably from 52 to 90 mass%, and particularly preferably from 55 to 80 mass%) per 100 mass% of a total amount of the structural units constituting the copolymer.

[Appendix 5] The texture improver according to any one of Appendices 2 to 4, wherein the hydrophobic monomer (B) contains a (meth)acrylic acid ester (preferably a (meth)acrylic acid alkyl ester).

[Appendix 6] The texture improver according to any one of Appendices 2 to 5, wherein a content proportion of the structural unit (b) derived from the hydrophobic monomer (B) in the copolymer is from 0.1 to 64 mass% (preferably from 1 to 60 mass%, more preferably from 2 to 55 mass%, still more preferably from 5 to 50 mass%, yet still more preferably from 10 to 45 mass%, and particularly preferably from 20 to 45 mass%) per 100 mass% of the total amount of the structural units constituting the copolymer.

[Appendix 7] The texture improver according to any one of Appendices 2 to 6, wherein in the copolymer, a content proportion of a structural unit (e) derived from an additional monomer (E) is from 0 to 10 mass% (preferably from 0 to 8 mass%, and more preferably from 0 to 5 mass%) per 100 mass% of the total amount of the structural units constituting the copolymer.

[Appendix 8] The texture improver according to any one of Appendices 2 to 7, wherein in the copolymer, a total content proportion of the structural unit (a) derived from the primary to tertiary amino group-containing monomer (A) and the structural unit (b) derived from the hydrophobic monomer (B) is 60 mass% or more (preferably 70 mass% or more, more preferably 80 mass% or more, and still more preferably 90 mass% or more) per 100 mass% of the total amount of the structural units constituting the copolymer.

[Appendix 9] The texture improver according to any one of Appendices 2 to 8, wherein a weight average molecular weight of the copolymer is from 4000 to 1000000 (preferably from 4500 to 800000, more preferably from 5000 to 600000, still more preferably from 6000 to 400000, yet still more preferably from 7000 to 200000, yet still more preferably from 10000 to 100000, and particularly preferably from 20000 to 80000).

[Appendix 10] The texture improver according to any one of Appendices 2 to 9, wherein the polyalkyleneimine is polyethyleneimine.

[Appendix 11] The texture improver according to any one of Appendices 2 to 10, wherein a weight average molecular weight of the polyalkyleneimine is 400 or more (preferably 1000 or more, more preferably 10000 or more, and still more preferably 50000 or more).

[Appendix 12] The texture improver according to any one of Appendices 2 to 11, wherein the weight average molecular weight of the polyalkyleneimine is 1000000 or less (preferably 800000 or less, more preferably 600000 or less, and still more preferably 500000 or less).

[Appendix 13] The texture improver according to any one of Appendices 2 to 12, wherein an amine value per nonvolatile content of the polyalkyleneimine is 5 or more (preferably 10 or more, and more preferably 15 or more).

[Appendix 14] The texture improver according to any one of Appendices 2 to 13, wherein an amine value per nonvolatile content of the polyalkyleneimine is 30 or less (preferably 25 or less, and more preferably 20 or less).

[Appendix 15] The texture improver according to any one of Appendices 2 to 14, wherein a cationization degree of the polyalkyleneimine is 5 meq/g or more (preferably 10 meq/g or more, and more preferably 15 meq/g or more).

[Appendix 16] The texture improver according to any one of Appendices 2 to 15, wherein the cationization degree of the polyalkyleneimine is 30 meq/g or less (preferably 25 meq/g or less, and more preferably 22 meq/g or less).

[Appendix 17] The texture improver according to any one of Appendices 2 to 16, wherein a molar ratio of a primary amine, a secondary amine, and a tertiary amine in the polyalkyleneimine is, in terms of primary amine : secondary amine : tertiary amine, from 10 to 50 : from 10 to 60 : from 10 to 50 (preferably from 20 to 45 : from 20 to 55 : from 10 to 40, and more preferably from 25 to 40 : from 30 to 50 : from 20 to 35).

[Appendix 18] The texture improver according to any one of Appendices 2 to 17, wherein a content proportion of the primary amine in the polyalkyleneimine is 10 mol% or more (preferably 20 mol% or more, more preferably 25 mol% or more, and still more preferably 27 mol% or more) per 100 mol% of a total amount of alkyleneimines constituting the polyalkyleneimine.

[Appendix 19] The texture improver according to any one of Appendices 2 to 18, wherein the content proportion of the primary amine in the polyalkyleneimine is 50 mol% or less (preferably 45 mol% or less, more preferably 40 mol% or less, and still more preferably 37 mol% or less) per 100 mol% of the total amount of the alkyleneimines constituting the polyalkyleneimine.

[Appendix 20] The texture improver according to any one of Appendices 2 to 19, wherein a content proportion of the secondary amine in the polyalkyleneimine is 10 mol% or more (preferably 20 mol% or more, and more preferably 30 mol% or more) per 100 mol% of the total amount of the alkyleneimines constituting the polyalkyleneimine.

[Appendix 21] The texture improver according to any one of Appendices 2 to 20, wherein the content proportion of the secondary amine in the polyalkyleneimine is 60 mol% or less (preferably 55 mol% or less, and more preferably 50 mol% or less) per 100 mol% of the total amount of the alkyleneimines constituting the polyalkyleneimine.

[Appendix 22] The texture improver according to any one of Appendices 2 to 21, wherein a content proportion of the tertiary amine in the polyalkyleneimine is 10 mol% or more (preferably 15 mol% or more, and more preferably 20 mol% or more) per 100 mol% of the total amount of the alkyleneimines constituting the polyalkyleneimine.

[Appendix 23] The texture improver according to any one of Appendices 2 to 22, wherein the content proportion of the tertiary amine in the polyalkyleneimine is 50 mol% or less (preferably 40 mol% or less, and more preferably 35 mol% or less) per 100 mol% of the total amount of the alkyleneimines constituting the polyalkyleneimine.

[Appendix 24] The texture improver according to any one of Appendices 2 to 23, wherein the polyalkyleneimine is a modified polyalkyleneimine.

[Appendix 25] A degree of branching of the polyalkyleneimine is more than 0% (preferably 1% or more, more preferably 5% or more, still more preferably 10% or more, and particularly preferably 15% or more).

[Appendix 26] The texture improver according to any one of Appendices 2 to 25, wherein the degree of branching of the polyalkyleneimine is 50% or less (preferably 40% or less, and more preferably 35% or less).

[Appendix 27] An inorganic powder having the primary to tertiary amino group-containing polymer compound described in any one of Appendices 1 to 26 present on a surface of the inorganic powder.

[Appendix 28] An inorganic powder having the texture improver described in any one of Appendices 1 to 26 present on a surface of the inorganic powder.

[Appendix 29] A composition, containing a primary to tertiary amino group-containing polymer compound and an inorganic powder.

[Appendix 30] A composition, containing the texture improver described in any one of Appendices 1 to 26 and an inorganic powder.

[Appendix 31] The composition according to Appendix 30, containing the inorganic powder and the texture improver present on a surface of the inorganic powder.

[Appendix 32] The composition according to Appendix 30 or 31, wherein a content proportion of the inorganic powder in the composition is from 0.5 to 60 mass% (preferably from 1 to 40 mass%, and more preferably from 5 to 20 mass%) per 100 mass% of a total amount of the composition.

[Appendix 33] The composition according to any one of Appendices 30 to 32, wherein a content proportion of the inorganic powder in the composition is from 70 to 99.99 mass% (preferably from 80 to 99.97 mass%, and more preferably from 90 to 99.95 mass%) per 100 mass% of the total amount of the composition.

[Appendix 34] The composition according to any one of Appendices 30 to 33, wherein a content of the primary to tertiary amino group-containing polymer compound in the composition is from 0.5 to 300 parts by mass (preferably from 1 to 250 parts by mass, more preferably from 2 to 200 parts by mass, and still more preferably from 3 to 150 parts by mass) per 100 parts by mass of a total amount of the inorganic powder.

[Appendix 35] The composition according to any one of Appendices 30 to 34, wherein a content of the primary to tertiary amino group-containing polymer compound in the composition is from 0.01 to 200 parts by mass (preferably from 0.03 to 100 parts by mass, more preferably from 0.05 to 50 parts by mass, still more preferably from 0.07 to 15 parts by mass, and particularly preferably from 0.08 to 5 parts by mass) per 1000 parts by mass of the total amount of the inorganic powder.

[Appendix 36] The composition according to any one of Appendices 30 to 35, wherein a zeta potential of the composition is positive (preferably 1 mV or higher, more preferably 5 mV or higher, still more preferably 10 mV or higher, and particularly preferably 20 mV or higher).

[Appendix 37] The composition according to any one of Appendices 30 to 36, wherein the zeta potential of the composition is negative (-5 mV or lower, -10 mV or lower, -20 mV or lower, or -30 mV or lower).

[Appendix 38] The composition according to any one of Appendices 30 to 37, wherein a solids content concentration of the composition is from 1 to 50 mass% (preferably from 5 to 30 mass%, and more preferably from 7 to 20 mass%) per 100 mass% of the total amount of the composition.

[Appendix 39] The composition according to any one of Appendices 30 to 38, wherein a solids content concentration of the composition is 90 mass% or more (95 mass% or more, 98 mass% or more, 99 mass% or more, or 95.5 mass% or more) per 100 mass% of the total amount of the composition.

[Appendix 40] The composition according to any one of Appendices 30 to 39, wherein a content proportion of a solvent (particularly, water) in the composition is 60 mass% or more (preferably 70 mass% or more, and more preferably 80 mass% or more) per 100 mass% of the total amount of the composition.

[Appendix 41] The composition according to Appendix 29, wherein the primary to tertiary amino group-containing polymer compound contains the primary to tertiary amino group-containing polymer compound described in any one of Appendices 2 to 9.

[Appendix 42] The composition according to any one of Appendices 29 to 41, which is a cosmetic composition.

[Appendix 43] A method for producing an inorganic powder, the inorganic powder having the texture improver described in any one of Appendices 1 to 26 present on a surface of the inorganic powder, the method including a mixing step of mixing the texture improver and an inorganic powder in a solvent.

[Appendix 44] The production method according to Appendix 43, further including a step of removing the solvent after the mixing step.

[Appendix 45] The production method according to Appendix 43 or 44, further including a pulverization step of pulverizing the inorganic powder after the mixing step.

## Claims

1. A texture improver, comprising a primary to tertiary amino group-containing polymer compound.

2. The texture improver according to claim 1, wherein the primary to tertiary amino group-containing polymer compound a copolymer containing a structural unit (a) derived from a primary to tertiary amino group-containing monomer (A) and a structural unit (b) derived from a hydrophobic monomer (B)comprises:; and/or a polyalkyleneimine.

3. The texture improver according to claim 2, wherein the hydrophobic monomer (B) comprises a (meth)acrylic acid ester.

4. The texture improver according to claim 2, wherein the polyalkyleneimine is polyethyleneimine.

5. An inorganic powder, comprising the texture improver described in any one of claims 1 to 4 present on a surface of the inorganic powder.

6. A composition, comprising the texture improver described in any one of claims 1 to 4 and an inorganic powder.

7. A composition, comprising a primary to tertiary amino group-containing polymer compound and an inorganic powder.

8. The composition according to claim 7, wherein the primary to tertiary amino group-containing polymer compound comprises: a copolymer containing a structural unit (a) derived from a primary to tertiary amino group-containing monomer (A) and a structural unit (b) derived from a hydrophobic monomer (B); and/or a polyalkyleneimine.

9. The composition according to claim 8, wherein the hydrophobic monomer (B) comprises a (meth)acrylic acid ester.

10. The composition according to claim 8, wherein the polyalkyleneimine is polyethyleneimine.

11. The composition according to any one of claims 7 to 10, which is a cosmetic composition.

12. A method for producing an inorganic powder, the inorganic powder comprising the texture improver described in any one of claims 1 to 4 present on a surface of the inorganic powder, the method comprising a mixing step of mixing the texture improver and an inorganic powder in a solvent.

13. The production method according to claim 12, further comprising a step of removing the solvent after the mixing step.

14. The production method according to claim 12, further comprising a pulverization step of pulverizing the inorganic powder after the mixing step.
